(19) Europäisches Patentamt
European Patent Office
Office européen des brevets

(11) Veröffentlichungsnummer: **0 010 249**
**A2**

(12) # EUROPÄISCHE PATENTANMELDUNG

(21) Anmeldenummer: **79103854.0**

(22) Anmeldetag: **08.10.79**

(51) Int. Cl.³: **C 07 C 143/74**
C 07 C 147/10, C 07 C 147/12
C 07 C 147/14, C 07 C 149/425
C 07 C 161/02, A 01 N 41/04
A 01 N 41/10, A 01 N 47/28
A 01 N 47/48
//C07C79/35, C07C93/14,
C07C147/06, C07C149/32,
C07C149/36, C07C149/42

(30) Priorität: **23.10.78 DE 2845996**

(43) Veröffentlichungstag der Anmeldung:
**30.04.80 Patentblatt 80/9**

(84) Benannte Vertragsstaaten:
**AT BE CH DE FR GB IT NL SE**

(71) Anmelder: **BAYER Aktiengesellschaft**
**Zentralbereich Patente,Marken und Lizenzen**
**Bayerwerk**
**D-5090 Leverkusen 1(DE)**

(72) Erfinder: **Kühle, Engelbert, Dr.**
**von-Bodelschwingh-Strasse 42**
**D-5060 Bergisch-Gladbach(DE)**

(72) Erfinder: **Klauke, Erich, Dr.**
**Eichendorffweg 8**
**D-5068 Odenthal(DE)**

(72) Erfinder: **Eue, Ludwig, Dr.**
**Paul-Klee-Strasse 36**
**D-5090 Leverkusen(DE)**

(72) Erfinder: **Schmidt, Robert R., Dr.**
**Hahnenweg 5**
**D-5000 Köln 80(DE)**

(54) **Herbizide Mittel, Verfahren zu ihrer Herstellung und ihre Verwendung zur Bekämpfung von Unkräutern.**

(57) Sulfonanilide der Formel

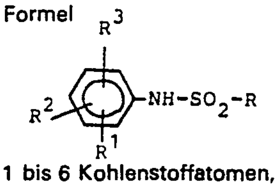

(I)

in welcher

R für Alkyl mit 1 bis 6 Kohlenstoffatomen, Chloralkyl mit 1 bis 6 Kohlenstoffatomen und bis zu 6 Chloratomen, gegebenenfalls substituierten Aryl oder für die Gruppierung der Formel

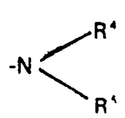

steht, worin

$R^4$ und $R^5$ gleich oder verschieden sind und für Alkyl mit 1 bis 6 Kohlenstoffatomen stehen oder

$R^4$ und $R^5$ gemeinsam mit dem angrenzenden Stickstoffatom für einen gesättigten oder ungesättigten heterocyclischen Ring stehen, der auch weitere Heteroatome enthalten kann,

$R^1$ für Alkoxy mit 1 bis 5 Kohlenstoffatomen, gegebenenfalls substituiertes Phenoxy, Alkylthio mit 1 bis 6 Kohlenstoffatomen, Alkenylthio mit bis zu 6 Kohlenstoffatomen, Alkylsulfonylaminoalkylthio mit 1 bis 6 Kohlenstoffatomen in der Alkylsulfonyl-Gruppe und 1 bis 6 Kohlenstoffatomen in der Alkylthio-Gruppe, gegebenenfalls substituiertes Phenylthio, Thiocyanato oder die Gruppierung der Formel $-S(O)_nR^6$ steht, worin

$R^6$ für Alkyl mit 1 bis 6 Kohlenstoffatomen, Halogenalkyl mit 1 bis 6 Kohlenstoffatomen und bis zu 6 Halogenatomen, gegebenenfalls substituiertes Phenyl oder gegebenenfalls substituiertes Benzyl steht und n für 1 oder 2 steht,

$R^2$ für Wasserstoff, Halogen, Alkyl mit 1 bis 6 Kohlenstoffatomen, Halogenalkyl mit 1 bis 6 Kohlenstoffatomen und bis zu 6 Halogenatomen steht, jedoch nicht für 2-Trifluormethyl steht, wenn R für Methyl oder Chlormethyl steht und gleichzeitig $R^1$ für paraständiges Methylthio, Methylsulfinyl oder Methylsulfonyl steht, und

$R^2$ weiterhin für Nitro oder die Gruppierung der Formel $-NH-CO-NH-R^7$ steht, worin $R^7$ für Alkyl mit 1 bis 6 Kohlenstoffatomen steht, und

$R^3$ für Wasserstoff oder Halogen steht,

besitzen starke herbizide Eigenschaften.

EP 0 010 249 A2

BAYER AKTIENGESELLSCHAFT 5090 Leverkusen, Bayerwerk
Zentralbereich Dü/AB
Patente, Marken und Lizenzen IIb

# Herbizide Mittel, Verfahren zu ihrer Herstellung und ihre Verwendung zur Bekämpfung von Unkräutern

Die vorliegende Erfindung betrifft die Verwendung von bestimmten Sulfonaniliden als Herbizide.

Es ist bereits bekannt geworden, daß zahlreiche Fluoralkyl-sulfonanilide herbizide Eigenschaften besitzen (vgl. US-PS 3 639 474, DE-OS 2 118 190, DE-OS 2 551 027 und DE-OS 2 703 477). So läßt sich zum Beispiel Methansulfonsäure-2-trifluormethyl-4-methylthio-anilid zur Bekämpfung von Unkraut verwenden. Dieser Wirkstoff befriedigt allerdings nicht immer hinsichtlich der Unkrautwirkung und auch nicht in der Selektivität gegenüber Kulturpflanzen.

Es wurde nun gefunden, daß die Sulfonanilide der Formel

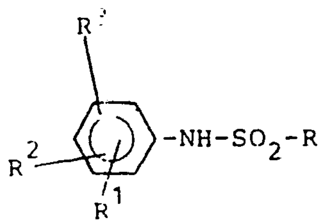

(I)

in welcher

R für Alkyl mit 1 bis 6 Kohlenstoffatomen, Chloralkyl mit 1 bis 6 Kohlenstoffatomen und bis zu 6 Chloratomen, gegebenenfalls substituierten Aryl oder für die Gruppierung der Formel

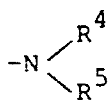 steht, worin

$R^4$ und $R^5$ gleich oder verschieden sind und für Alkyl mit 1 bis 6 Kohlenstoffatomen stehen oder

$R^4$ und $R^5$ gemeinsam mit dem angrenzenden Stickstoffatom für einen gesättigten oder ungesättigten heterocyclischen Ring stehen, der auch weitere Heteroatome enthalten kann,

$R^1$ für Alkoxy mit 1 bis 6 Kohlenstoffatomen, gegebenenfalls substituiertes Phenoxy, Alkylthio mit 1 bis 6 Kohlenstoffatomen, Alkenylthio mit bis zu 6 Kohlenstoffatomen, Alkylsulfonylaminoalkylthio mit 1 bis 6 Kohlenstoffatomen in der Alkylsulfonyl-Gruppe und 1 bis 6 Kohlenstoffatomen in der Alkylthio-Gruppe, gegebenenfalls substituiertes Phenylthio, Thiocyanato oder für die Gruppierung der Formel $-S(O)_nR^6$ steht, worin

$R^6$ für Alkyl mit 1 bis 6 Kohlenstoffatomen, Halogenalkyl

mit 1 bis 6 Kohlenstoffatomen und bis zu 6 Halogenatomen, gegebenenfalls substituiertes Phenyl oder gegebenenfalls substituiertes Benzyl steht und

n für 1 oder 2 steht,

$R^2$ für Wasserstoff, Halogen, Alkyl mit 1 bis 6 Kohlenstoffatomen, Halogenalkyl mit 1 bis 6 Kohlenstoffatomen und bis zu 6 Halogenatomen steht, jedoch nicht für 2-Trifluormethyl steht, wenn R für Methyl oder Chlormethyl steht und gleichzeitig $R^1$ für para-ständiges Methylthio, Methylsulfinyl oder Methylsulfonyl steht, und

$R^2$ weiterhin für Nitro oder die Gruppierung der Formel $-NH-CO-NH-R^7$ steht, worin $R^7$ für Alkyl mit 1 bis 6 Kohlenstoffatomen steht, und

$R^3$ für Wasserstoff oder Halogen steht,

sehr gut zur Unkrautbekämpfung geeignet sind.

Überraschenderweise zeigen die erfindungsgemäß verwendbaren Sulfonanilide der Formel (I) neben einer guten Unkrautwirkung vor allem eine sehr gute Wirksamkeit gegen Cyperus-Arten, die mit vorbekannten, konstitutionell ähnlichen Sulfonaniliden nicht immer ausreichend bekämpfbar sind. In der Regel benötigt man zur Bekämpfung von Cyperus-Arten relativ hohe Aufwandmengen an bekannten Herbiziden, diese können dann aber nicht mehr zur selektiven Bekämpfung von Cyperus Arten in Kulturen eingesetzt werden. Die erfindungsgemäß verwendbaren Stoffe stellen somit eine wertvolle Bereicherung der Technik dar.

Die erfindungsgemäß verwendbaren Sulfonanilide sind durch die Formel (I) allgemein definiert. In dieser Formel steht R vorzugsweise für geradkettiges oder verzweigtes Alkyl mit 1 bis 4 Kohlenstoffatomen, Chloralkyl mit 1 bis 4 Kohlenstoffatomen und bis zu 5 Chloratomen. Weiterhin steht R vorzugsweise für gegebenenfalls durch Alkyl mit 1 bis 4 Kohlenstoffatomen, Halogen, Halogenalkyl mit 1 bis 4 Kohlenstoffatomen und bis zu 5 Halogenatomen, insbesondere Fluor und/oder Chloratomen, und/oder Nitro substituiertes Aryl mit 6 bis 10 Kohlenstoffatomen. Außerdem steht R vorzugsweise für die Gruppierung

$-N\begin{smallmatrix}R^4\\R^5\end{smallmatrix}$, worin $R^4$ und $R^5$ unabhängig voneinander vorzugsweise für Alkyl mit 1 bis 4 Kohlenstoffatomen stehen, oder $R^4$ und $R^5$ gemeinsam mit dem angrenzenden Stickstoffatom vorzugsweise für einen gesättigten oder ungesättigten heterocyclischen Ring mit 5 oder 6 Ringgliedern stehen, der außer dem Stickstoffatom noch Sauerstoff, Schwefel und/oder weitere Stickstoffatome als Heteroatome enthalten kann.

Als heterocyclische Reste seien Pyrrolidinyl, Piperidinyl, Hexamethylenimidinyl, Morpholinyl und Thiamorpholinyl beispielhaft genannt. $R^1$ steht in der Formel (I) vorzugsweise für Alkoxy mit 1 bis 4 Kohlenstoffatomen, gegebenenfalls durch Alkyl mit 1 bis 4 Kohlenstoffatomen,



BAD ORIGINAL

Alkylthio mit 1 bis 4 Kohlenstoffatomen, Alkylsulfonyl mit 1 bis 4 Kohlenstoffatomen und/oder Halogen substituiertes Phenoxy, Alkylthio mit 1 bis 4 Kohlenstoffatomen, Alkenylthio mit bis zu 4 Kohlenstoffatomen, Alkylsulfonylamino-alkylthio mit 1 bis 4 Kohlenstoffatomen in der Alkylsulfonyl-Gruppe und 1 bis 4 Kohlenstoffatomen in der Alkylthio-Gruppe, gegebenenfalls durch Alkyl mit 1 bis 4 Kohlenstoffatomen, Alkylthio mit 1 bis 4 Kohlenstoffatomen und oder Halogen substituiertes Phenylthio, für Thiocyanato oder für die Gruppierung $-S(O)_nR^6$, worin n für 1 oder 2 steht und $R^6$ vorzugsweise für Alkyl mit 1 bis 4 Kohlenstoffatomen, Halogenalkyl mit bis zu 4 Kohlenstoffatomen und bis zu 5 gleichen oder verschiedenen Halogenatomen, insbesondere Fluor- oder Chloratomen, für gegebenenfalls durch Alkyl mit 1 bis 4 Kohlenstoffatomen und/oder Halogen, insbesondere Fluor oder Chlor, substituiertes Phenyl oder für gegebenenfalls durch Alkyl mit 1 bis 4 Kohlenstoffatomen und/oder Halogen, insbesondere Fluor oder Chlor, substituiertes Benzyl steht. $R^2$ steht in der Formel (I) vorzugsweise für Wasserstoff, Fluor, Chlor, Brom, geradkettiges oder verzweigtes Alkyl mit 1 bis 4 Kohlenstoffatomen, Halogenalkyl mit bis zu 4 Kohlenstoffatomen und bis zu 5 gleichen oder verschiedenen Halogenatomen, insbesondere Fluor oder Chloratomen, jedoch nicht für 2-Trifluormethyl, wenn R für Methyl oder Chlormethyl steht und $R^1$ gleichzeitig für para-ständiges Methylthio, Methylsulfinyl oder Methylsulfonyl steht. $R^2$ steht weiterhin vorzugsweise für Nitro oder die Gruppierung der Formel $-NH-CO-NH-R^7$, worin $R^7$ vorzugsweise für geradkettiges oder verzweigtes Alkyl mit 1 bis 4 Kohlenstoff-

atomen steht. $R^3$ steht in der Formel (I) vorzugsweise für Wasserstoff, Fluor, Chlor oder Brom.

Als Beispiele für erfindungsgemäß verwendbare Sulfonanilide seien im einzelnen die folgenden Verbindungen der Formel (I) genannt:

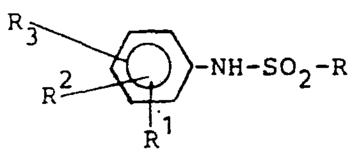

(I)

| R | $R^1$ | $R^2$ | $R^3$ |
|---|---|---|---|
| $CH_3$ | 4-$SO_2CH_3$ | 3 Cl | H |
| $C_2H_5$ | 4-$SO_2CH_3$ | 3 Cl | H |
| n-$C_3H_7$ | 4-$SO_2CH_3$ | 3 Cl | H |
| i-$C_3H_7$ | 4-$SO_2CH_3$ | 3 Cl | H |
| $C_2H_3Cl_2$ | 4-$SO_2CH_3$ | 3 Cl | H |
| 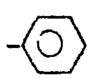 | 4-$SO_2CH_3$ | 3 Cl | H |
| 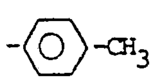 | 4-$SO_2CH_3$ | 3 Cl | H |
| 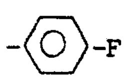 | 4-$SO_2CH_3$ | 3 Cl | H |
| 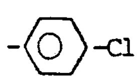 | 4-$SO_2CH_3$ | 3 Cl | H |

(Fortsetzung)

| R | $R^1$ | $R^2$ | $R^3$ |
|---|---|---|---|
| 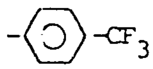 | $4-SO_2CH_3$ | 3 Cl | H |
| 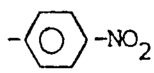 | $4-SO_2CH_3$ | 3 Cl | H |
| 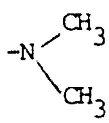 | $4-SO_2CH_3$ | 3 Cl | H |
| 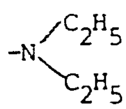 | $4-SO_2CH_3$ | 3 Cl | H |
| $-N(C_3H_7)_2$ | $4-SO_2CH_3$ | 3 Cl | H |
| 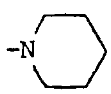 | $4-SO_2CH_3$ | 3 Cl | H |
| 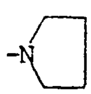 | $4-SO_2CH_3$ | 3 Cl | H |
| 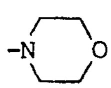 | $4-SO_2CH_3$ | 3 Cl | H |
| 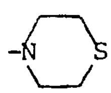 | $4-SO_2CH_3$ | 3 Cl | H |

(Fortsetzung)

| R | $R^1$ | $R^2$ | $R^3$ |
|---|---|---|---|
| $-CH_2Cl$ | $4-OC_2H_5$ | 3 Cl | H |
| $-CH_2Cl$ | $4-OC_3H_7$ | 3 Cl | H |
| $-CH_3$ | 4-O-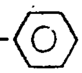-Cl | 3 Cl | H |
| $-CH_3$ | 4-O-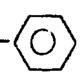$-CH_3$ | 3 $CF_3$ | H |
| $-CH_2Cl$ | 4-O-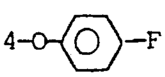-F | 3 Cl | H |
| $-CH_2Cl$ | 4-O-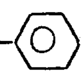$-SCH_3$ | 3 Cl | H |
| $-CH_2Cl$ | 4-O-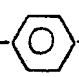$-SO_2CH_3$ | 3 Cl | H |
| $-CH_2Cl$ | $4-S-C_2H_5$ | 2 $CF_3$ | H |
| $-CH_2Cl$ | $4-S-C_3H_7$ | 2 $CF_3$ | H |
| $-CH_2Cl$ | $4-SO_2CH_3$ | H | H |
| $-CH_2Cl$ | $4-SO_2CH_3$ | 3 Cl | H |
| $-CH_2Cl$ | $2-S-CH_2-CH=CH-CH_3$ | H | H |
| $-C_2H_5$ | $2-S-CH_2-CH_2-NH-SO_2CH_3$ | H | H |

(Fortsetzung)

| R | $R^1$ | $R^2$ | $R^3$ |
|---|---|---|---|
| $-CH_3$ | $2-S-CH_2-CH_2-NH-SO_2C_2H_5$ | H | H |
| $-CH_2Cl$ | 4-S- 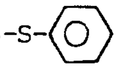 $-CH_3$ | $2-CF_3$ | H |
| $-CH_2Cl$ | 4-S- 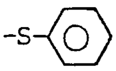 -Cl | H | H |
| $-CH_3$ | 4-SCN | H | H |
| $-CH_3$ | 4-SCN | 2 $CF_3$ | H |
| $-CH_2Cl$ | $4-SO_2CF_3$ | 3 Cl | H |
| $-CH_2-Cl$ | $4-SO_2CF_3$ | 5 Cl | 2 Cl |
| $-CH_3$ | $4-SO_2-$ 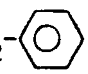 $-CH_3$ | 2 $CF_3$ | H |
| $-CH_3$ | $4-SO_2-$ 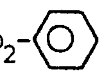 -F | 2 $CF_3$ | H |
| $-CH_2Cl$ | $2-SO_2-CH_2-$ 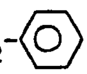 -Cl | H | H |
| $-CH_2Cl$ | $2-SO_2-CH_2-$ 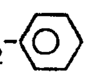 -F | H | H |
| $-CH_2Cl$ | $4-SO_2CH_3$ | 2 F | 4 |
| $-CH_2Cl$ | $5-SO_2CH_3$ | 4 $NO_2$ | 2 Cl |
| $-CH_2Cl$ | $5-SO_2C_2H_5$ | 4 $NO_2$ | H |
| $-CH_2Cl$ | $5-SO_2CH_2Cl$ | 4-NH-CO-NH-$C_2H_5$ | 2 Cl |

Die erfindungsgemäß verwendbaren Sulfonanilide der Formel (I) sind bisher noch nicht bekannt. Sie lassen sich jedoch in einfacher Weise nach im Prinzip bekannten Verfahren herstellen. So erhält man

a) Sulfonanilide der Formel (I), indem man Aniline der Formel

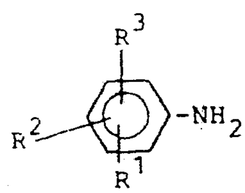

(II)

in welcher
$R^1$, $R^2$ und $R^3$ die oben angegebene Bedeutung haben,

mit Sulfochloriden der Formel

$$Cl-SO_2-R \quad (III)$$

in welcher
R die oben angegebene Bedeutung hat,

in Gegenwart eines Säureakzeptors sowie gegebenenfalls in Gegenwart eines Verdünnungsmittels umsetzt,

b) diejenigen Sulfonanilide der Formel (I), in denen $R^1$ für die Gruppierung der Formel $-S(O)_nR^6$ steht, worin $R^6$ und n die oben angegebene Bedeutung haben, indem man Sulfonanilide der Formel

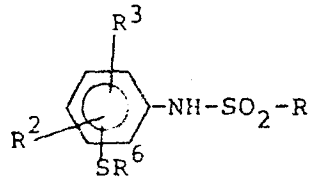

(Ia)

in welcher
R, $R^2$, $R^3$ und $R^6$ die oben angegebene Bedeutung haben,

gegebenenfalls in Gegenwart eines Verdünnungsmittels oxidiert,

c) diejenigen Verbindungen der Formel (I), in denen

R für Alkyl mit 1 bis 6 Kohlenstoffatomen oder Chloralkyl mit 1 bis 6 Kohlenstoffatomen und bis zu 6 Chloratomen steht,

$R^1$ für die Gruppierung $-S(O)_2R^8$ steht, wobei $R^8$ für Alkyl mit 1 bis 6 Kohlenstoffatomen oder Halogenalkyl mit bis zu 6 Kohlenstoffatomen und bis zu 6 Halogenatomen steht,

$R^2$ für Nitro steht und

$R^3$ für Wasserstoff oder Halogen steht,

in dem man Sulfonanilide der Formel

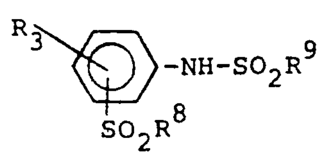

(IV)

in welcher
$R^3$ die oben angegebene Bedeutung hat,

$R^8$ für Alkyl mit 1 bis 6 Kohlenstoffatomen

oder Halogenalkyl mit bis zu 6 Kohlenstoffatomen und bis zu 6 Halogenatomen steht und

$R^9$ für Alkyl mit 1 bis 6 Kohlenstoffatomen oder Chloralkyl mit bis zu 6 Kohlenstoffatomen und bis zu 6 Chloratomen steht,

in Gegenwart von Schwefelsäure mit Salpetersäure nitriert,

d) diejenigen Verbindungen der Formel (I), in denen

R für Alkyl mit 1 bis 6 Kohlenstoffatomen oder Chloralkyl mit 1 bis 6 Kohlenstoffatomen und bis zu 6 Chloratomen steht,

$R^1$ für die Gruppierung $-S(O)_2R^8$ steht, wobei $R^8$ die oben angegebene Bedeutung hat,

$R^2$ für die Gruppierung $-NH-CO-NH-R^7$ steht, worin $R^7$ die oben angegebene Bedeutung hat, und

$R^3$ die oben angegebene Bedeutung hat,

indem man Nitro-Verbindungen der Formel

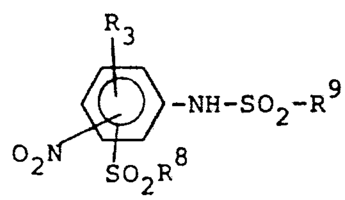

(V)

in welcher
$R^3$, $R^8$ und $R^9$ die oben angegebene Bedeutung haben,

in Gegenwart eines Katalysators sowie in Gegenwart eines Verdünnungsmittels mit Wasserstoff hydriert und die dabei entstehenden Verbindungen der Formel

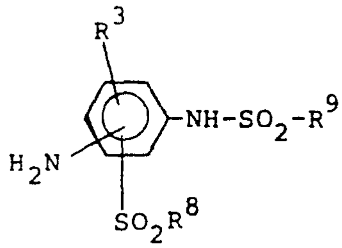

(VI)

in welcher
$R^3$, $R^8$ und $R^9$ die oben angegebene Bedeutung haben,

mit Isocyanaten der Formel

$$R^7\text{-NCO} \qquad (VII)$$

in welcher
$R^7$ die oben angegebene Bedeutung hat,
in Gegenwart eines Verdünnungsmittels umsetzt.

Die erfindungsgemäß verwendbaren Sulfonanilide der Formel (I) sowie deren Herstellung nach den Verfahren (a) bis (d) sind Gegenstand eines gesonderten Schutzbegehrens.

Verwendet man 3-Chlor-4-ethylmercapto-anilin und Methansulfochlorid als Ausgangsstoffe, so läßt sich der Verlauf des Verfahrens (a) durch das folgende Formelschema wiedergeben:

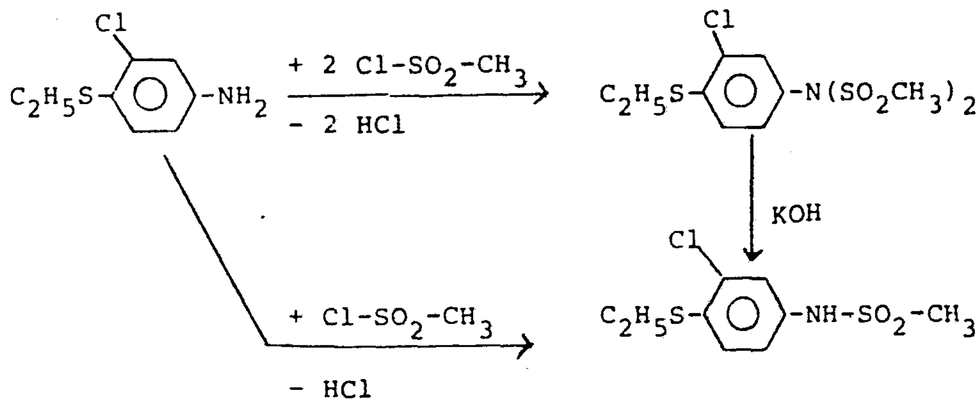


Verwendet man 3-Chlor-4-ethylmercapto-methan-sulfonanilid als Ausgangsstoff und Wasserstoffperoxid als Oxidationsmittel, so läßt sich der Verlauf des Verfahrens (b) durch das folgende Formelschema wiedergeben:

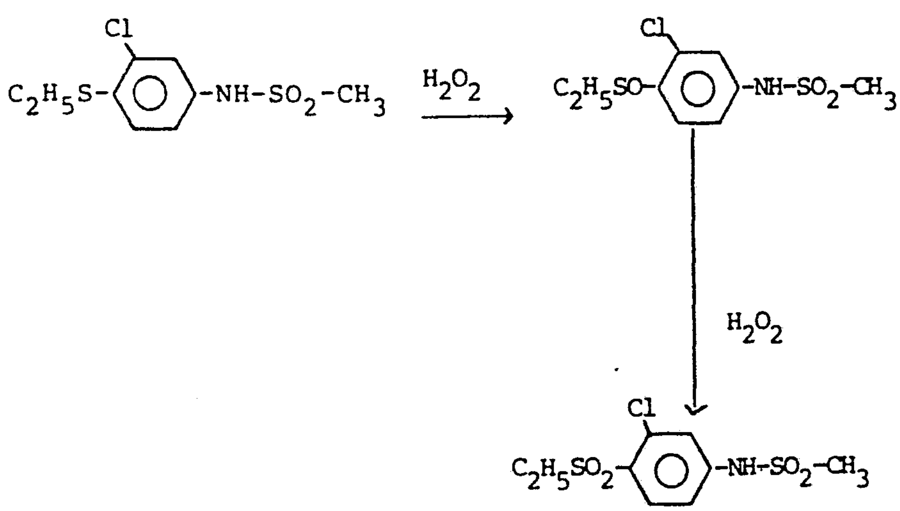

Verwendet man 2-Chlor-5-ethylsulfonyl-methansulfonanilid als Ausgangsstoff und ein Gemisch aus Salpetersäure und Schwefelsäure als Nitrierungsmittel, so läßt sich der Verlauf des Verfahrens (c) durch das folgende Formelschema wiedergeben:

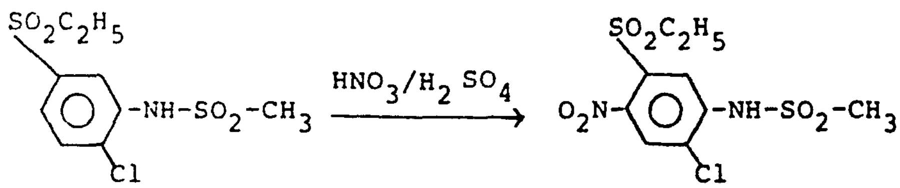


Verwendet man 2-Chlor-4-nitro-5-ethylsulfonyl-methansulfonanilid als Ausgangsstoff, Wasserstoff in Gegenwart von Raney-Nickel als Hydrierungsmittel und Ethylisocyanat als Reaktionskomponente, so läßt sich der Verlauf des Verfahrens (d) durch das folgende Formelschema wiedergeben:

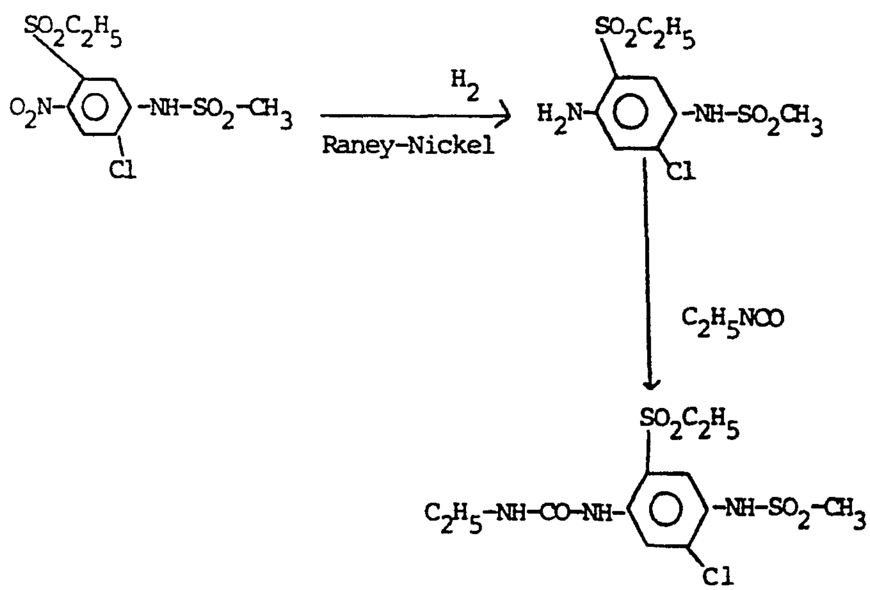

Die bei dem Verfahren (a) als Ausgangsstoffe zu verwendenden Aniline sind durch die Formel (II) allgemein definiert. In dieser Formel stehen $R^1$, $R^2$ und $R^3$ vorzugsweise für diejenigen Reste, die bereits im Zusammenhang mit der Beschreibung der erfindungsgemäß verwendbaren Sulfonanilide der Formel (I) vorzugsweise für diese Reste genannt wurden.

Als Beispiele für Aniline der Formel (II) seien im einzelnen aufgeführt:

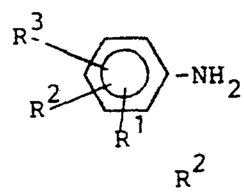 (II)

| $R^1$ | $R^2$ | $R^3$ |
|---|---|---|
| 5-$SO_2C_2H_5$ | 2 Cl | H |
| 4-$SO_2C_2H_5$ | 3 Cl | H |
| 4-$SOC_2H_5$ | 3 Cl | H |
| 4-$SOC_3H_7$ | 3 Cl | H |
| 4-$SC_2H_5$ | 3 Cl | H |
| 5-$SC_2H_5$ | 2 Cl | H |
| 4-$OCH_3$ | 3 Cl | H |
| 4-$OCH_3$ | H | H |
| 4-$OC_3H_7$ | 3 Cl | H |
| 4-O- 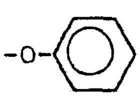 | H | H |
| 4-O- 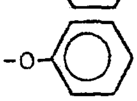 | 3 Cl | H |

Fortsetzung

| $R^1$ | $R^2$ | $R^3$ |
|---|---|---|
| 4-O-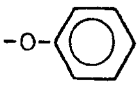-$CH_3$ | 3 $CF_3$ | H |
| 4-O-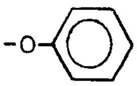-F | 3 Cl | H |
| 4-O-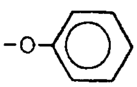-$SCH_3$ | 3 Cl | H |
| 4-O-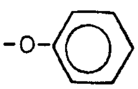-$SO_2CH_3$ | 3 Cl | H |
| 4-O-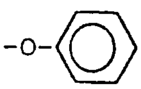-$SOCH_3$ | 3 Cl | H |
| 4-S-$C_2H_5$ | 2 $CF_3$ | H |
| 4-S-$C_3H_7$ | 2 $CF_3$ | H |
| 2-S-$CH_2$-CH=CH-$CH_3$ | H | H |
| 2-S-$CH_2$-$CH_2$NH-$SO_2CH_3$ | H | H |
| 2-S-$CH_2$-$CH_2$-NH-$SO_2$-$C_2H_5$ | H | H |
| 4-S-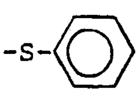-$CH_3$ | 2 $CF_3$ | H |
| 4-S-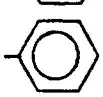-Cl | H | H |
| 4-SCN | H | H |
| 4-SCN | 2 $CF_3$ | H |

Fortsetzung

| $R^1$ | $R^2$ | $R^3$ |
|---|---|---|
| $4-SO_2C_2H_5$ | 5 Cl | 2 Cl |
| $4-SO_2-$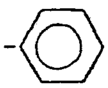$-CH_3$ | $2\ CF_3$ | H |
| $4-SO_2-$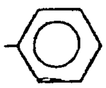$-F$ | $2\ CF_3$ | H |
| $2-SO_2-CH_2-$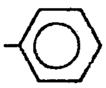$-Cl$ | H | H |
| $2-SO_2-CH_2-$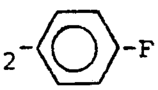$-F$ | H | H |
| $5-SO_2C_2H_5$ | $2\ CF_3$ | H |
| $5-SO_2C_2H_5$ | $4\ NO_2$ | 2 Cl |

Die Aniline der Formel (II) sind bekannt oder lassen sich nach im Prinzip bekannten Verfahren herstellen (vgl. z.B. Houben-Weyl, "Methoden der organischen Chemie", Band IX, Seite 105 ff; 4. Auflage 1955 sowie DE-OS 2 551 027).

Man erhält Aniline der Formel (II) zum Beispiel, indem man

α) Nitroverbindungen der Formel

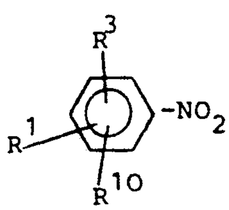

(VIII)

in welcher
$R^1$ und $R^3$ die oben angegebene Bedeutung haben und

$R^{10}$ für Wasserstoff, Halogen, Alkyl mit 1 bis 6 Kohlenstoffatomen oder Halogenalkyl mit 1 bis 6 Kohlenstoffatomen und bis zu 6 Halogenatomen steht

mit Wasserstoff in Gegenwart eines Katalysators, wie Raney-Nickel oder Cobaltsulfid, sowie in Gegenwart eines Verdünnungsmittels, wie z.B. Methanol, bei Temperaturen zwischen 20 °C und 150°C reduziert,

oder indem man

ß) Amino-thiophenole der Formel

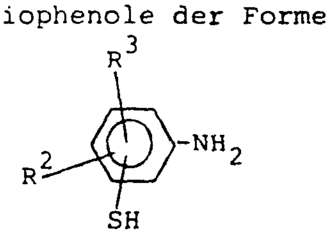

(IX)

in welcher
$R^2$ und $R^3$ die oben angegebene Bedeutung haben,

mit Halogenverbindungen der Formel

$R^{11}$-Hal (X)

in welcher

$R^{11}$ für Alkyl mit 1 bis 6 Kohlenstoffatomen,

Alkenyl mit bis zu 6 Kohlenstoffatomen, gegebenenfalls substituiertes Phenyl oder gegebenenfalls substituiertes Benzyl steht und

Hal für Chlor oder Brom steht,

in Gegenwart eines Verdünnungsmittels, wie Methylenchlorid sowie gegebenenfalls in Gegenwart eines Säurebindemittels, wie Pyridin, bei Temperaturen zwischen 0 °C und 120 °C umsetzt, und gegebenenfalls anschließend oxidiert,

oder indem man

γ) Isocyanate der Formel

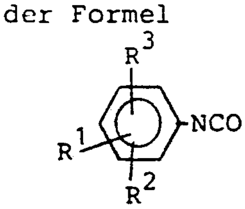

(XI)

in welcher
$R^1$, $R^2$ und $R^3$ die oben angegebene Bedeutung haben,

mit starken Säuren, wie zum Beispiel Schwefelsäure, in Gegenwart eines Verdünnungsmittels, wie zum Beispiel Chlorbenzol, bei Temperaturen zwischen 20 °C und 100 °C behandelt.

Die Verbindungen der Formeln (VIII) bis (XI) sind bekannt oder lassen sich nach im Prinzip bekannten Verfahren herstellen (vgl. nach Herstellungsbeispiele).



BAD ORIGINAL

Die bei dem Verfahren (a) weiterhin als Ausgangsstoffe benötigten Sulfochloride sind durch die Formel (III) allgemein definiert. In dieser Formel hat R vorzugsweise diejenigen Bedeutungen, die bereits im Zusammenhang mit der Beschreibung der erfindungsgemäß verwendbaren Sulfonanilide der Formel (I) vorzugsweise für den Rest R genannt wurden.

Als Beispiele für Sulfochloride der Formel (III) seien im einzelnen genannt:

Methansulfochlorid
Ethansulfochlorid
Propansulfochlorid
Chlormethansulfochlorid
Trifluormethansulfochlorid
Phenyl-sulfochlorid
4-Methyl-phenyl-sulfochlorid
4-Fluor-phenyl-sulfochlorid
4-Chlor-phenyl-sulfochlorid
4-Trifluormethyl-phenyl-sulfochlorid
4-Nitro-phenyl-sulfochlorid
Dimethylamino-sulfochlorid
Diethylamino-sulfochlorid
Di-n-propyl-amino-sulfochlorid
N-Chlorsulfonyl-piperidin
N-Chlorsulfonyl-pyrrolidin
N-Chlorsulfonyl-hexamethylenimin
N-Chlorsulfonyl-morpholin

N-Chlorsulfonyl-thiamorpholin

Die Sulfochloride der Formel (III) sind bekannt oder lassen sich nach im Prinzip bekannten Verfahren herstellen.

Die bei dem Verfahren (b) als Ausgangsstoffe zu verwendenden Sulfonanilide sind durch die Formel (Ia) allgemein definiert. In dieser Formel haben R, $R^2$, $R^3$ und $R^6$ vorzugsweise diejenigen Bedeutungen, die bereits im Zusammenhang mit der Beschreibung der erfindungsgemäß verwendbaren Sulfonanilide der Formel (I) vorzugsweise für diese Reste genannt wurden.

Die Sulfonanilide der Formel (Ia) sind bislang noch nicht bekannt. Sie lassen sich jedoch nach dem Verfahren (a) in einfacher Weise herstellen.

Als Oxidationsmittel kommen bei der Durchführung des Verfahrens (b) alle üblichen sauerstoff-abgebenden Oxidationsmittel in Frage. Hierzu gehören vorzugsweise Wasserstoffperoxid, Peressigsäure und m-Chlorperbenzoesäure.

Die bei dem Verfahren (c) als Ausgangsstoffe zu verwendenden Sulfonanilide sind durch die Formel (IV) allgemein definiert. In dieser Formel hat $R^3$ vorzugsweise diejenigen Bedeutungen, die bereits im Zusammenhang mit der Beschreibung der erfindungsgemäß verwend-



BAD ORIGINAL

baren Sulfonanilide der Formel (I) vorzugsweise für diesen Rest genannt wurden. $R^8$ steht vorzugsweise für Alkyl mit 1 bis 4 Kohlenstoffatomen oder für Halogenalkyl mit bis zu 4 Kohlenstoffatomen und bis zu 5 gleichen oder verschiedenen Halogenatomen, insbesondere Fluor- oder Chloratomen. $R^9$ steht vorzugsweise für Alkyl mit 1 bis 4 Kohlenstoffatomen oder für Chloralkyl mit bis zu 4 Kohlenstoffatomen und bis zu 5 Chloratomen.

Die Sulfonanilide der Formel (IV) sind bisher noch nicht bekannt. Sie lassen sich jedoch nach den Verfahren (a) bzw. (b) in einfacher Weise herstellen.

Als Nitrierungsmittel wird bei der Durchführung des Verfahrens (c) ein Gemisch aus Schwefelsäure und Salpetersäure (Nitriersäure) verwendet.

Die bei dem Verfahren (d) als Ausgangsstoffe zu verwendenden Nitro-substituierten Sulfonanilide sind durch die Formel (V) allgemein definiert. In dieser Formel hat $R^3$ vorzugsweise diejenigen Bedeutungen, die bereits im Zusammenhang mit der Beschreibung der erfindungsgemäß verwendbaren Sulfonanilide der Formel (I) vorzugsweise für diesen Rest genannt wurden.

$R^8$ und $R^9$ haben vorzugsweise diejenigen Bedeutungen, die bereits im Zusammenhang mit der Beschreibung der Verbindungen der Formel (IV) vorzugsweise für diese Reste genannt wurden.

Die Nitro-substituierten Sulfonanilide der Formel (V) sind bisher noch nicht bekannt. Sie lassen sich jedoch in einfacher Weise nach dem Verfahren (c) herstellen.

Die bei dem Verfahren (d) als Reaktionskomponenten benötigten Isocyanate sind durch die Formel (VII) allgemein definiert. In dieser Formel steht $R^7$ vorzugsweise für diejenigen Reste, die bereits im Zusammenhang mit der Beschreibung der erfindungsgemäß verwendbaren Sulfonanilide der Formel (I) vorzugsweise für diesen Rest genannt wurden.

Als Beispiele für Isocyanate der Formel (VII) seien im einzelnen genannt:
Methylisocyanat
Ethylisocyanat
Propylisocyanat
n-Butylisocyanat.

Die Isocyanate der Formel (VII) sind bekannt.

Bei der Durchführung des Verfahrens (a) können als Säureakzeptoren alle üblichen Säurebindemittel fungieren. Hierzu gehören vorzugsweise Alkalihydroxide wie Natriumhydroxid und Kaliumhydroxid, ferner Alkalicarbonate wie Natriumcarbonat und Kaliumcarbonat, außerdem Alkalialkohole wie Natriummethylat und Natriumethylat, weiterhin aliphatische, aromatische oder heterocyclische Amine wie beispielsweise Triethylamin, Dimethylamin, Dimethylanilin, Dimethylbenzylamin und Pyridin.

Als Verdünnungsmittel können bei der Umsetzung nach dem Verfahren (a) alle inerten organischen Lösungsmittel verwendet werden. Hierzu gehören vorzugsweise aliphatische oder aromatische Kohlenwasserstoffe, wei Petrolether, Benzin, Benzol, Toluol und Xylol, ferner chlorierte aliphatische oder aromatische Kohlenwasserstoffe, wie Methylenchlorid, Chloroform, Tetrachlorkohlenstoff und Chlorbenzol, weiterhin Ether, wie Diethylether, Dibutylether, Tetrahydrofuran und Dioxan, und außerdem Ketone, wie Aceton. Darüber hinaus kann auch Wasser als Verdünnungsmittel eingesetzt werden.

Die Reaktionstemperaturen können bei dem Verfahren (a) innerhalb eines größeren Bereiches variiert werden. Im allgemeinen arbeitet man die Temperaturen zwischen $0^{o}C$ und $100^{o}C$, vorzugsweise zwischen $10^{o}C$ und $50^{o}C$.

Bei der Durchführung des Verfahrens (a) setzt man auf 1 Mol Anilin der Formel (II) 1 bis 3 Mol an Sulfochlorid der Formel (III) ein. Bei Verwendung von Sulfochlorid in mehr als der äquimolaren Menge kann auch das zweite Wasserstoffatom am Stickstoff des Anilins durch den Rest $-SO_2R$ substituiert werden. Letztere Verbindungen lassen sich jedoch durch Behandlung mit starken Basen, wie z.B. Kaliumhydroxid, in das gewünschte Produkt überführen. Die Isolierung der Reaktionsprodukte erfolgt nach üblichen Methoden. Im allgemeinen geht man so vor, daß man das Reaktionsgemisch mit einer verdünnten starken Säure versetzt. Dabei scheidet sich das Reaktionsprodukt in vielen Fällen kristallin ab. Ist im Reaktionsprodukt ein größerer

Anteil an solchen Substanzen vorhanden, in denen das Stickstoffatom des eingesetzten Anilins durch 2 - $SO_2R$ Gruppen substituiert ist, so empfiehlt es sich, nach dem Versetzen mit verdünnter Säure anschließend mit einem mit Wasser nicht mischbaren organischen Lösungsmittel zu extrahieren, dann das Lösungsmittel zu entfernen, den verbleibenden Rückstand mit einer starken Base zu behandeln und anschließend anzusäuern.

Bei der Durchführung des Verfahrens (b) können als Verdünnungsmittel alle üblicherweise für derartige Oxidationen verwendbaren inerten organischen Lösungsmittel eingesetzt werden. Hierzu gehören vorzugsweise Eisessig und Methylenchlorid.

Die Reaktionstemperaturen können bei der Oxidation gemäß Verfahren (b) innerhalb eines größeren Bereiches variiert werden. Im allgemeinen arbeitet man bei Temperaturen zwischen $0^{o}C$ und $120^{o}C$, vorzugsweise zwischen $50^{o}C$ und $100^{o}C$.

Bei der Durchführung des Verfahrens (b) setzt man auf 1 Mol an Sulfonanilid der Formel (Ib) eine äquimolare Menge oder einen Überschuß an Oxidationsmittel ein, und zwar in Abhängigkeit davon, ob die Sulfinyl- oder die Sulfonyl-Verbindung der Formel (I) synthetisiert werden soll. Die Aufarbeitung erfolgt nach üblichen Methoden. Im allgemeinen verfährt man in der Weise, daß man nach beendeter Umsetzung mit Wasser versetzt. Dabei scheidet sich das Reaktionsprodukt häufig unmittelbar in kristalliner Form ab. Ist dies nicht der Fall, so extrahiert man das Reaktionsgemisch mit einem



BAD ORIGINAL

mit Wasser nicht mischbaren organischen Lösungsmittel, trennt die Phasen und entfernt das Lösungsmittel.

Bei der Umsetzung gemäß Verfahren (c) arbeitet man im allgemeinen ohne zusätzliches Lösungsmittel.

Die Reaktionstemperaturen können bei dem Verfahren (c) innerhalb eines größeren Bereiches variiert werden. Im allgemeinen arbeitet man bei Temperaturen zwischen 0°C und 60°C, vorzugsweise zwischen 10°C und 50°C.

Bei der Durchführung des Verfahrens (c) setzt man die Sulfonanilide der Formel (IV) mit einem Überschuß an Nitrierungsmittel um. Die Aufarbeitung erfolgt nach üblichen Methoden.

Bei der Umsetzung gemäß Verfahren (d) arbeitet man sowohl bei der Durchführung der ersten Stufe als auch bei der Durchführung der zweiten Stufe in Gegenwart eines Verdünnungsmittels. In der ersten Stufe können alle üblicherweise für derartige Hydrierungen verwendbaren organischen Lösungsmittel eingesetzt werden. Hierzu gehören vorzugsweise Alkohole, wie Methanol und Ethanol, ferner Ether, wie Tetrahydrofuran und Dioxan.

In der zweiten Stufe kommen als Verdünnungsmittel alle inerten organischen Lösungsmittel in Betracht. Hierzu gehören vorzugsweise Ether, wie Diethylether und Dioxan, ferner Kohlenwasserstoffe, wie Benzol und Toluol, Chlorkohlenwasserstoffe, wie Methylenchlorid und Chlorbenzol, weiterhin Ketone, wie Aceton und Cyclohexanon.

Bei der Durchführung der ersten Stufe des Verfahrens (d) kommen als Katalysatoren alle für derartige Hydrierungen üblicherweise verwendbaren Katalysatoren in Frage. Hierzu gehören vorzugsweise Raney-Nickel und Cobaltsulfid.

Bei der in der ersten Stufe des Verfahrens (d) ablaufenden Hydrierung kann der Wasserstoffdruck innerhalb eines größeren Bereiches variiert werden. Im allgemeinen arbeitet man bei Drucken zwischen 1 bar und 150 bar, vorzugsweise zwischen 20 bar und 50 bar.

Die Reaktionstemperaturen können bei dem Verfahren (d) sowohl in der ersten als auch in der zweiten Stufe innerhalb eines größeren Bereichs variiert werden. Im allgemeinen arbeitet man in der ersten Stufe bei Temperaturen zwischen 0 °C und 100 °C, vorzugsweise zwischen 10 °C und 50°C. In der zweiten Stufe arbeitet man im allgemeinen bei Temperaturen zwischen 20°C und 100°C, vorzugsweise zwischen 40°C und 80°C.

Bei der Durchführung des Verfahrens (d) werden in der ersten Stufe Nitro-Verbindungen der Formel (V) mit einem Überschuß an Wasserstoff hydriert. Die Isolierung der Reaktionsprodukte erfolgt nach üblichen

Methoden. In der zweiten Stufe des Verfahrens (d) setzt man auf 1 Mol an einer Verbindung der Formel (VI) 1 Mol bis 1,5 Mol an Isocyanat der Formel (VII) ein. Die Aufarbeitung erfolgt auch hierbei nach üblichen Methoden.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cuburbita.

Monokotyle Unkräuter der Gattungen: Echinochloa, Setaria, Paricum, Digitaria, Phleum, Poa, Festuca, Eleusine,

Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäß verwendbaren Sulfonanilide zeigen nicht nur eine ausgezeichnete Unkrautwirkung, sondern sind darüber hinaus auch zur selektiven Unkrautbekämpfung geeignet. Sie lassen sich zum Beispiel zur selektiven Unkrautbekämpfung in Baumwolle, Getreide, Mais, Soja, Reis, Rüben und Gemüsekulturen verwenden.



BAD ORIGINAL

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone wie Aceton, Methyläthylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,1 und 10 kg Wirkstoff pro ha, vorzugsweise zwischen 0,1 und 5 kg/ha.



BAD ORIGINAL

Die gute herbizide Wirksamkeit der erfindungsgemäß verwendbaren Sulfonanilide geht aus dem folgenden Beispiel hervor.

Beispiel A

Pre-emergence-Test

Lösungsmittel: 5 Gewichtsteile Aceton
Emulgator: 1 Gewichtsteil Alkylarylpolyglycolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Wirkstoffe, Aufwandmengen und Resultate gehen aus der nachfolgenden Tabelle hervor:

Tabelle, Beispiel A

Pre emergence-Test

| Wirkstoff +) | Wirkstoffaufwand kg/ha | Echinochloa | Chenopodium | Matricaria | Galinsoga | Stellaria | Lolium | Avena fatua | Cyperus | Weizen | Baumwolle |
|---|---|---|---|---|---|---|---|---|---|---|---|
| ( 35 ) | 5,0 | 80 | 80 | 100 | 90 | 80 | 100 | 80 | 100 | 80 | 0 |
| | 2,5 | 80 | 80 | 80 | 80 | 60 | 100 | 80 | 100 | 80 | 0 |
| ( 14 ) | 5,0 | - | 40 | 60 | 60 | 80 | 60 | - | 100 | 0 | 0 |
| | 2,5 | - | 0 | 20 | 20 | 80 | 60 | - | 80 | 0 | 0 |
| ( 15 ) | 5,0 | 80 | 60 | 60 | 80 | 90 | 80 | 80 | 100 | 60 | 0 |
| | 2,5 | 80 | 60 | 40 | 60 | 90 | 80 | 60 | 100 | 60 | 0 |
| ( 36 ) | 5,0 | 80 | 40 | 90 | 90 | 100 | 90 | 80 | 100 | 80 | 0 |
| | 2,5 | 80 | 20 | 80 | 80 | 80 | 80 | 80 | 100 | 70 | 0 |

+) vgl. Wirkstoffliste auf der folgenden Seite

0010249



Wirkstoffliste

( 35 ) = 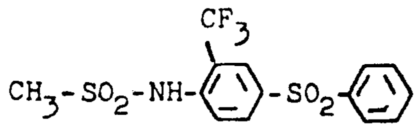

( 14 ) = 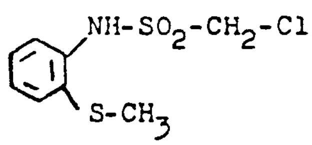

( 15 ) = 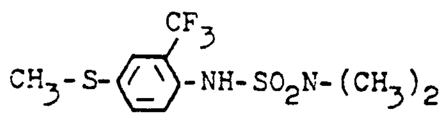

( 36 ) = 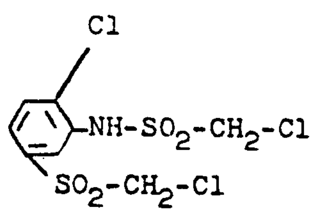

Herstellungsbeispiele

Beispiel 1

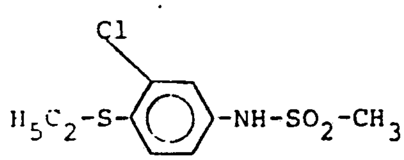

Eine Lösung von 13 g (70 mMol) 3-Chlor-4-ethylmercapto-anilin in 100 ml Pyridin wird bei $0^{\circ}C$ bis $5^{\circ}C$ unter Rühren tropfenweise mit 8,5 g (74 mMol) Methansulfochlorid versetzt. Man rührt noch etwa 30 Minuten bei Raumtemperatur, gießt dann das Reaktionsgemisch in verdünnte wäßrige Salzsäure und saugt den ausfallenden Feststoff ab. Dieses Produkt wird in verdünnter wäßriger Natronlauge gelöst. Man behandelt die Lösung mit Aktivkohle, filtriert und säuert mit verdünnter wäßriger Salzsäure an. Dabei fällt das Reaktionsprodukt in kristalliner Form aus. Man erhält auf diese Weise 15 g (81,5 % der Theorie) an 3-Chlor-4-ethylmercapto-methylsulfonanilid vom Schmelzpunkt 115-116°C.

In analoger Weise werden die in der nachfolgenden Tabelle 1 formelmäßig aufgeführten Sulfonanilide der Formel (I) hergestellt.

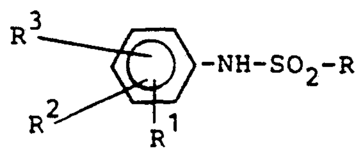

(I)

Tabelle 1



| Beispiel-Nr. | R | $R^1$ | $R^2$ | $R^3$ | Schmelzpunkt [°C] bzw. Brechungsindex |
|---|---|---|---|---|---|
| 2 | $CH_2Cl$ | 4-$OCH_3$ | 3 Cl | H | 125-126 |
| 3 | $CH_2Cl$ | 4-SCN | 3 Cl | H | 110-113 |
| 4 | $CH_2Cl$ | 4-O-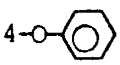-Cl | 2 $CF_3$ | H | 97-99 |
| 5 | $CH_3$ | 4-S-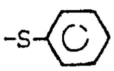 | 2 $CF_3$ | H | 63-65 |
| 6 | $CH_3$ | 4-O-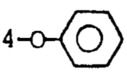-Cl | 3 $CF_3$ | H | 125-128 |
| 7 | $CH_2Cl$ | 2-S-$CH_2$-CH=$CH_2$ | H | H | $n_D^{20}$ = 1,5902 |
| 8 | $CH_2Cl$ | 4-O-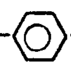-$SCH_3$ | 2 $CF_3$ | H | 107-108 |
| 9 | $CH_2Cl$ | 2-S-$C_2H_5$ | H | H | 43-45 |
| 10 | $CH_3$ | 4-$SCH_3$ | 3 $CF_3$ | H | 132-133 |
| 11 | $CH_2Cl$ | 4-$SCH_3$ | H | H | 120-122 |
| 12 | $CH_2Cl$ | 4-$SC_2H_5$ | 3 Cl | H | 98-100 |
| 13 | $CH_2Cl$ | 2-$SC_3H_7$-i | H | H | $n_D^{20}$ 1,5730 |
| 14 | $CH_2Cl$ | 2-$SCH_3$ | H | H | $n_D^{20}$ 1,6007 |
| 15 | -$N(CH_3)_2$ | 4-$SCH_3$ | 2 $CF_3$ | H | $n_D^{20}$ 1,5399 |
| 16 | -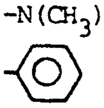 | 4-$SCH_3$ | 2 $CF_3$ | H | 98-99 |
| 17 | $CH_3$ | 4-$SO_2CF_2Cl$ | 3 Cl | H | 163-165 |
| 18 | $CH_3$ | 4-$SO_2CH_3$ | 3 Cl | H | 93 |
| 19 | $CH_3$ | 4-$SOCH_3$ | 3 Cl | H | 120-123 |
| 20 | $CH_2Cl$ | 4-$SO_2CH_3$ | 3 Cl | H | 139-141 |
| 21 | $CH_2Cl$ | 4-$SOCH_3$ | 3 Cl | H | 185-188 |
| 22 | $CH_2Cl$ | 3-$SO_2CH_3$ | 4 Cl | H | 180-181 |
| 23 | $CH_3$ | 4-$SO_2CF_3$ | H | H | 163-165 |

Tabelle 1 (Fortsetzung)

| Beispiel-Nr. | R | $R^1$ | $R^2$ | $R^3$ | Schmelzpunkt [°C] bzw. Brechungsindex |
|---|---|---|---|---|---|
| 24 | $CH_3$ | 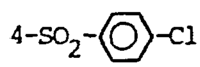 | 2 $CF_3$ | H | 173-177 |
| 25 | $CH_3$ | 4-$SO_2$-$C_4H_9$ | 2 Cl | 5 Cl | 137-138 |
| 26 | $CH_2Cl$ | 3-$SO_2$-$C_2H_5$ | 6 Cl | H | 127-128 |
| 27 | $CH_2Cl$ | 4-$SO_2CF_2Cl$ | H | H | 146-149 |
| 28 | $CH_3$ | 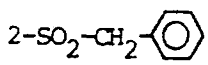 | H | H | 145-146 |
| 29 | $CH_2Cl$ | 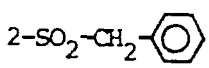 | H | H | 155-156 |

Beispiel 30

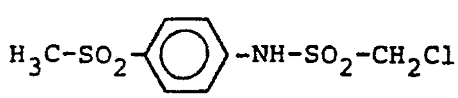

17 g Methyl-(4-aminophenyl)-sulfon werden unter Zusatz von 30 ml Triethylamin in 100 ml Dioxan gelöst und tropfenweise mit 30 g Chlormethansulfochlorid versetzt. Die Temperatur steigt hierbei auf etwa 75°C an. Nach dem Abkühlen trägt man das Reaktionsprodukt in Wasser ein, saugt das dabei entstehende kristalline Produkt ab und löst es in methanolisch-wäßriger Natronlauge. Man filtriert und fällt das Reaktionsprodukt durch Zugabe von wäßriger Salzsäure aus. Fp.: 175-177°C; Ausbeute: 10 g = 35 % d. Th.

In analoger Weise werden die in der nachfolgenden Tabelle 2 formelmäßig aufgeführten Sulfonanilide der Formel (I) hergestellt.

Tabelle 2

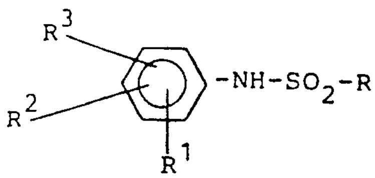

(I)

| Beispiel-Nr. | R | $R^1$ | $R^2$ | $R^3$ | Schmelzpunkt [°C] bzw. Brechungsindex |
|---|---|---|---|---|---|
| 31 | $CH_2Cl$ | 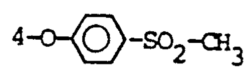 | 2 $CF_3$ | H | 166-167 |
| 32 | $-N(CH_3)_2$ | 4-$SO_2CH_3$ | 2 $CF_3$ | H | 122-123 |
| 33 | $CH_3$ | 4-$SO_2$-$C_2H_5$ | 3 Cl | H | 154-156 |
| 34 | $CH_2Cl$ | 4-$SO_2C_2H_5$ | 3 Cl | H | 110-113 |
| 35 | $CH_3$ | 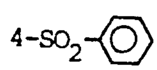 | 2 $CF_3$ | H | 173-174 |

Beispiel 36

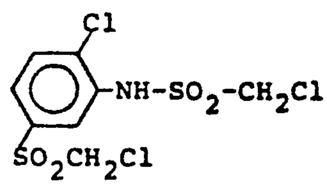

120 g 2-Chlor-5-chlormethansulfonyl-anilin werden in 500 ml Pyridin gelöst und unter Kühlung bei 0-5°C tropfenweise mit 150 g Chlormethansulfochlorid versetzt. Man rührt eine weitere Stunde und gießt dann das Reaktionsgemisch in verdünnte wäßrige Salzsäure. Das ausfallende Produkt wird in verdünnter wäßriger Natronlauge gelöst. Man behandelt die Lösung mit Aktivkohle, filtriert und fügt verdünnte wäßrige Salzsäure hinzu. Der ausgefallene Feststoff wird abgesaugt und aus Methanol umkristallisiert. Man erhält auf diese Weise 100 g (56 % der Theorie) des Reaktionsproduktes vom Schmelzpunkt 145-146°C.

Beispiel 37

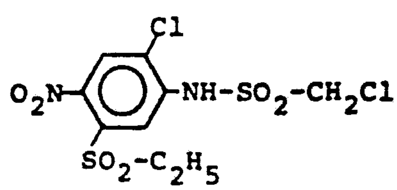

a) 300 g 2-Chlor-5-ethylsulfonylanilin werden in 1 Liter Pyridin gelöst und unter Kühlung bei 0-5°C tropfenweise mit 190 g Chlormethan-sulfochlorid versetzt. Man rührt noch eine weitere Stunde und arbeitet dann nach der im Beispiel 39 angegebenen Methode auf. Man erhält auf

diese Weise 247 g (55 % der Theorie) an 2-Chlor-5-ehtylsulfonyl-chlormethansulfonanilid vom Schmelzpunkt 127-129°C.

b) 70 g 2-Chlor-5-ethylsulfonyl-chlormethan-sulfonanilid werden in 200 ml konzentrierter Schwefelsäure gelöst und tropfenweise mit 15 ml konzentrierter Salpetersäure versetzt. Die Temperatur steigt dabei auf etwa 50°C an. Danach wird das Reaktionsgemisch auf Eis gegeben. Der anfallende Feststoff wird mit Wasser gewaschen und aus Methanol umkristallisiert. Man erhält auf diese Weise 65 g (82 % der Theorie) an 2-Chlor-4-nitro-5-ethyl-sulfonyl-chlormethylsulfonanilid vom Schmelzpunkt 185-187°C.

Beispiel 38

Nach der im Beispiel 40 angegebenen Methode erhält man durch Nitrierung von 2-Chlor-5-chlormethylsulfonyl-chlormethyl-sulfonanilid die Verbindung der Formel

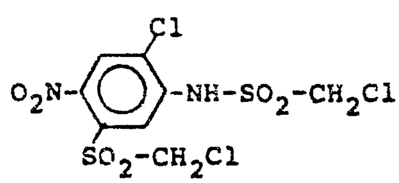

Fp = 189-191°C

Beispiel 39

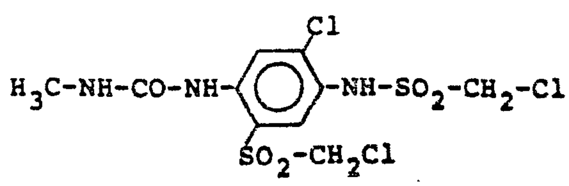

a) 2-Chlor-4-nitro-5-chlormethylsulfonyl-chlormethylsulfonanilid wird in Methanol gelöst und in Gegenwart von Cobaltsulfid mit Wasserstoff hydriert. Nach dem Aufarbeiten erhält man 2-Chlor-4-amino-5-chlormethylsulfonyl-chlormethylsulfonanilid in Form eines Feststoffes vom Schmelzpunkt 228-229°C.

b) 8 g 2-Chlor-4-amino-5-chlormethylsulfonyl-chlormethylsulfonanilid werden in 100 ml Dioxan gelöst und unter Zusatz einer katalytischen Menge Triethylamin bei 30-40°C mit 2 g Methylisocynanat umgesetzt. Man rührt noch eine kurze Zeit bei der genannten Temperatur und fügt dann Wasser hinzu. Das dabei anfallende Festprodukt wird abgesaugt. Man erhält auf diese Weise 4,5 g des Reaktionsproduktes in Form eines Feststoffes vom Schmelzpunkt 188-190°C.

Beispiel 40

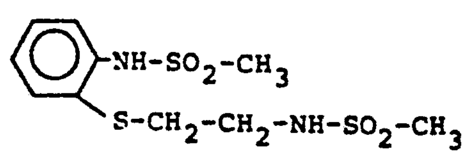

17 g 2-(ß-Aminoethylmercapto)-anilin werden in 100 ml Pyridin gelöst und bei 0-5°C mit 23 g Methansulfochlorid versetzt. Man rührt noch eine Weile nach und gießt dann das Reaktionsgemisch in verdünnte wäßrige Salzsäure. Das dabei anfallende Produkt wird in Methylenchlorid aufgenommen. Man trocknet und engt ein. Auf diese Weise erhält man 25 g (28 % der Theorie) des gewünschten Reaktionsproduktes in Form eines zähen Öles. Die Struktur der Substanz wurde mit Hilfe des Kernresonanz-Spektrums bestimmt.

Beispiel (II-1)

a)

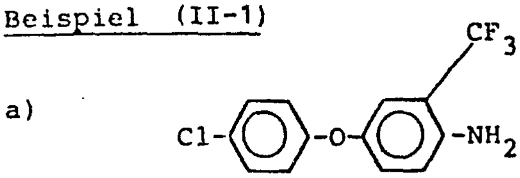

185 g 3-Trifluormethyl-4-nitro-4'-chlor-diphenylether werden in 900 ml Methanol gelöst und nach Zusatz von 30 g Raney-Nickel in einem Autoklaven wie 50°C unter einem Wasserstoffdruck von 55 bar hydriert.

Nach dem Abkühlen und Entspannen wird das Reaktionsgemisch filtriert und nach dem Abziehen des Lösungsmittels destilliert. Man erhält 158 g (93,5 % der Theorie) an 3-Trifluormethyl-4-amino-4'-chlor-diphenyläther.
Fp. 48-49°C.
$Kp_{0,1}$ 138-141°C

b)

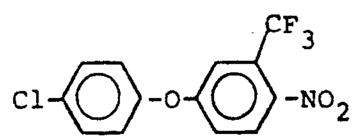

1 Mol 4-Chlorphenol und 56 g Kaliumhydroxid werden in 600 ml Dimethylsulfoxid vorgelegt und unter Rühren auf 130°C erwärmt. Bei dieser Temperatur werden 226 g (1 Mol) 2-Nitro-5-chlor-benzotrifluorid zugegeben. Anschließend wird der Ansatz 2 Stunden bei 145°C gerührt. Danach wird das Lösungsmittel unter vermindertem Druck abgezogen und der Rückstand auf Wasser gegeben. Die organische Phase wird abgetrennt. Der wäßrige Rückstand wird mit Methylenchlorid extrahiert. Man vereinigt die organischen Phasen, wäscht zweimal mit Wasser, trocknet über Natriumsulfat und destilliert. Nach einem Verlauf von unverändertem Ausgangsmaterial erhält man 187 g (59 % der Theorie) an 3-Trifluormethyl-4-nitro-4'-chlordiphenylether als Flüssigkeit die bei $Kp_{0,2}$: 159-161°C siedet.
$n_D^{20}$: 1,5670

In analoger Weise erhält man die nachstehend aufgeführten Aniline:

(II-2)

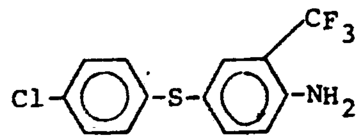

$Kp_{0,2}$: 152-154°C
Fp.: 64- 65°C

(II-3)

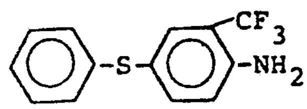

$Kp_{0,2}$: 132°C
Fp.: 51-53°C

(II-4)

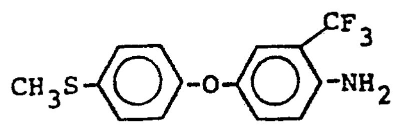

$Kp_{0,1}$: 145°C
Fp.: 48-50°C

(II-5)

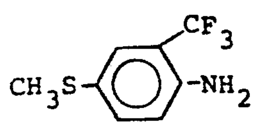

$Kp_{15}$: 126°C
$n_D^{20}$: 1,5482

(II-6)

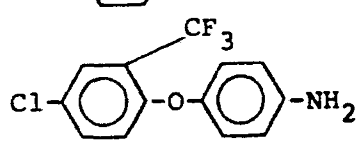

$Kp_{0,1}$: 145-147°C
$n_D^{20}$ : 1,5650

(II-7)

a)

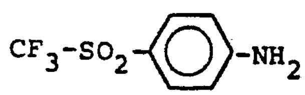

4-Nitro-phenyl-trifluormethylsulfon wird im Methanol gelöst und in einem Autoklaven in Gegenwart von Raney-Nickel als Katalysator bei 50°C unter einem Wasserstoff-Druck von 50 bar hydriert. Die Aufarbeitung erfolgt in der Weise, daß man das Reaktionsgemisch filtriert und dann einengt. Man erhält 4-Trifluormethylsulfonylanilin in 95 %iger Ausbeute in Form von Kristallen mit einem Schmelzpunkt von 94-95°C.

b)

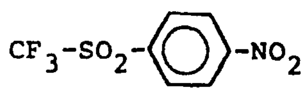

Eine Lösung von 53 g 4-Trifluormethylmercaptonitrobenzol in 100 ml Essigsäure wird bei Raumtemperatur unter Rühren in ein Gemisch aus 66 g Chromtrioxid in 100 ml Essigsäure getropft. Nach Ende der Zugabe erwärmt man auf 90°C und rührt bei dieser Temperatur noch 1 Stunde nach. Anschließend gibt man das Reaktionsgemisch auf Eis, extrahiert mit Methylenchlorid und zieht das Lösungsmittel ab. Man erhält 35 g 4-Nitro-phenyl-trifluormethylsulfon vom Schmelzpunkt 83-84°C.

Beispiel (II-8)

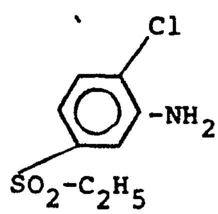

Nach der im Beispiel (II-7) angegebenen Methode erhält man ausgehend von 2-Chlor-5-ethylsulfonyl-nitro-benzol das 2-Chlor-5-ethylsulfonyl-anilin als Feststoff vom Schmelzpunkt 80-83°C.

(II-9)

a)

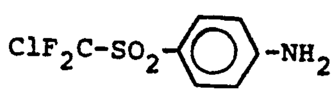

4-Nitrophenyl-difluorchlormethylsulfon wird in Methanol gelöst und in einem Autoklaven in Gegenwart von Raney-Nickel als Katalysator bei 50°C unter einem Wasserstoff-Druck von 50 bar hydriert. Die Aufarbeitung erfolgt in der

Weise, daß man das Reaktionsgemisch filtriert und dann einengt. Man erhält 4-Difluorchlormethylsulfonyl-anilin das zwischen 98-101°C schmilzt.

b)

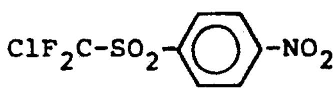

Eine Lösung von 4-Difluorchlormethyl-mercaptonitrobenzol in Essigsäure wird bei Raumtemperatur unter Rühren in ein Gemisch aus Chromtrioxid und Essigsäure getropft. Nach Ende der Zugabe erwärmt man auf 90°C und rührt bei dieser Temperatur noch 1 Stunde nach. Anschließend gibt man das Reaktionsgemisch auf Eis, extrahiert mit Methylenchlorid und zieht das Lösungsmittel ab. Man erhält 4-Difluorchlormethylsulfonyl-nitrobenzol vom Schmelzpunkt 101°C.

c)

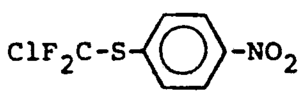

273 g 4-Trichlormethylmercapto-nitrobenzol werden vorgelegt und bei 0°C mit 200 ml Fluorwasserstoffsäure versetzt. Man läßt bei Raumtemperatur bis zu 20°C so lange reagieren, bis kein Fluor/Chlor-Austausch mehr erfolgt und die Chlorwasserstoff-Entwicklung beendet ist. Die überschüssige Fluorwasserstoffsäure wird unter vermindertem Druck abgezogen, und der Rückstand wird destilliert. Man erhält 221 g (92 % der Theorie) an 4-Difluorchlormethyl-mercapto-nitrobenzol.

$Kp_{0,4}$: 85°C

Fp.: 35-37°C.

(II-10)

a) 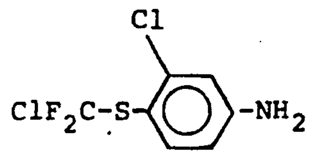

Eine Lösung von 200 g 3-Chlor-4-difluorchlormethylmercapto-phenylisocyanat in 200 ml Chlorbenzol wird bei 12-13°C unter intensivem Rühren in 320 ml konzentrierte Schwefelsäure getropft. Anschließend wird 30 Minuten bis zur Beendigung der Kohlendioxid-Entwicklung nachgeführt. Danach gießt man das Reaktionsgemisch auf Eis, saugt den kristallinen Niederschlag ab, versetzt ihn mit wäßriger Natronlauge und nimmt mit Toluol auf. Man destilliert das Toluol ab und erhält 150 g eines Rückstandes mit einem Brechungsindex $n_D^{20}$: 1,5927. Gemäß Gaschromatogramm beträgt der Gehalt an 3-Chlor-4-difluorchlormethylmercapto-anilin 97,2 %.

Die Reinigung des Produktes erfolgt durch Destillation.
$Kp_{0,3}$: 120°C

$n_D^{20}$ : 1,5925

b) 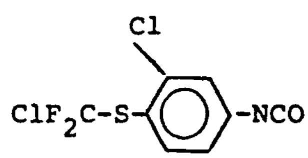

1880 g 3-Chlor-4-trichlormethylmercapto-phenylisocyanat werden bei 5-10°C innerhalb von 2 Stunden in 840 ml wasserfreie Flußsäure eingetropft. Dabei setzt eine heftige Chlorwasserstoff-Entwicklung ein.

Nach beendeter Zugabe läßt man das Gemüse bei 20°C noch weitere 8 Stunden reagieren bis sich kein Chlorwasserstoff mehr entwickelt. Danach wird die überschüssige Flußsäure abgezogen, und der Rückstand wird destilliert. Man erhält 1225 g an 3-Chlor-4-difluorchlormethylmercaptophenylisocyanat.

$Kp_{14}$: 139°C

$n_D^{20}$: 1,5650

Patentansprüche

1. Herbizide Mittel, gekennzeichnet durch einen Gehalt an mindestens einem Sulfonanilid der Formel

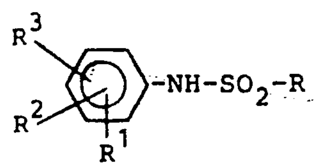

(I)

in welcher

R für Alkyl mit 1 bis 6 Kohlenstoffatomen, Chloralkyl mit 1 bis 6 Kohlenstoffatomen und bis zu 6 Chloratomen, gegebenenfalls substituiertes Aryl oder für die Gruppierung der Formel

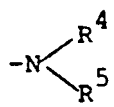 steht, worin

$R^4$ und $R^5$ gleich oder verschieden sind und für Alkyl mit 1 bis 6 Kohlenstoffatomen stehen oder

$R^4$ und $R^5$ gemeinsam mit dem angrenzenden Stickstoffatom für einen gesättigten oder ungesättigten heterocyclischen Ring stehen, der auch weitere Heteroatome enthalten kann,

$R^1$ für Alkoxy mit 1 bis 6 Kohlenstoffatomen, gegebenenfalls substituiertes Phenoxy, Alkylthio mit 1 bis 6 Kohlenstoffatomen, Alkenylthio mit bis zu 6 Kohlenstoffatomen, Alkylsulfonylaminoalkylthio mit 1 bis 6 Kohlenstoffatomen in der Alkylsulfonyl-Gruppe und 1 bis 6 Kohlenstoffatomen in der Alkylthio-Gruppe, gegebenenfalls

substituiertes Phenylthio, Thiocyanato oder die Gruppierung der Formel $-S(O)_nR^6$ steht,

worin

$R^6$ für Alkyl mit 1 bis 6 Kohlenstoffatomen, Halogenalkyl mit 1 bis 6 Kohlenstoffatomen und bis zu 6 Halogenatomen, gegebenenfalls substituiertes Phenyl oder gegebenenfalls substituiertes Benzyl steht und

n für 1 oder 2 steht,

$R^2$ für Wasserstoff, Halogen, Alkyl mit 1 bis 6 Kohlenstoffatomen, Halogenalkyl mit 1 bis 6 Kohlenstoffatomen und bis zu 6 Halogenatomen steht, jedoch nicht für 2-Trifluormethyl steht, wenn R für Methyl oder Chlormethyl steht und gleichzeitig $R^1$ für para-ständiges Methylthio, Methylsulfinyl oder Methylsulfonyl steht, und $R^2$ weiterhin für Nitro oder die Gruppierung der Formel $-NH-CO-NH-R^7$ steht, worin

$R^7$ für Alkyl mit 1 bis 6 Kohlenstoffatomen steht, und

$R^3$ für Wasserstoff oder Halogen steht.

2) Verfahren zur Bekämpfung von Unkräutern, dadurch gekennzeichnet, daß man Sulfonanilide der Formel (I) gemäß Anspruch 1 auf Unkräuter oder ihren Lebensraum einwirken läßt.

3) Verwendung von Sulfonaniliden der Formel (I) gemäß Anspruch 1 zur Bekämpfung von Unkräutern.

4) Verfahren zur Herstellung von herbiziden Mitteln, dadurch gekennzeichnet, daß man Sulfonanilide der Formel (I) gemäß Anspruch 1 mit Streckmitteln und/oder oberflächenaktiven Mitteln vermischt.